(19) Europäisches Patentamt
European Patent Office
Office européen des brevets

(11) Veröffentlichungsnummer: **0 173 204**
**A2**

(12) # EUROPÄISCHE PATENTANMELDUNG

(21) Anmeldenummer: **85110353.1**

(22) Anmeldetag: **19.08.85**

(51) Int. Cl.[4]: **C 07 D 401/14**, C 07 D 405/14, A 61 K 31/44

(30) Priorität: **30.08.84 DE 3431862**

(43) Veröffentlichungstag der Anmeldung: **05.03.86 Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Rosentreter, Ulrich, Dr., Kondorweg 23, D-5600 Wuppertal (DE)**
Erfinder: **Perzborn, Elisabeth, Dr., Am Tescherbusch 13, D-5600 Wuppertal 11 (DE)**
Erfinder: **Seuter, Friedel, Dr., Moospfad 16, D-5600 Wuppertal (DE)**

(54) **Neue 1,4-Dihydropyridine, Verfahren zu ihrer Herstellung sowie ihre Verwendung in Arzneimitteln.**

(57) Die vorliegende Erfindung betrifft doppelt basische Dihydropyridine, Verfahren zur ihrer Herstellung und ihre Verwendung in Arzneimitteln, insbesondere in antithrombotisch/antiischämisch wirksamen Arzneimitteln.

EP 0 173 204 A2

BAYER AKTIENGESELLSCHAFT 5090 Leverkusen, Bayerwerk
Konzernverwaltung RP
Patentabteilung KS/Ke-c

# Neue 1,4-Dihydropyridine, Verfahren zu ihrer Herstellung sowie ihre Verwendung in Arzneimitteln

Die vorliegende Erfindung betrifft neue 1,4-Dihydropyridine, Verfahren zu ihrer Herstellung und ihre Verwendung in Arzneimitteln, insbesondere in antithrombotisch/antiischämisch wirksamen Arzneimitteln.

Die vorliegende Erfindung betrifft neue 1,4-Dihydropyridine der allgemeinen Formel (I)

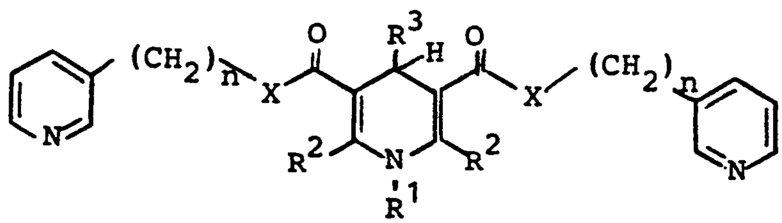

(I)

in der

$R^1$ für Wasserstoff oder Alkyl (geradkettig oder verzweigt), gegebenenfalls substituiert durch Halogen, Nitro, Cyano, Alkoxy, Carboxy, Alkoxycarbonyl oder Carboxamid steht,

$R^2$ für Wasserstoff oder Alkyl (geradkettig oder verzweigt), steht,

$R^3$ für Wasserstoff, Alkyl (geradkettig oder verzweigt), Carboxy, Alkoxycarbonyl oder Aryl, gegebenenfalls substituiert durch: Hydroxyalkyl, Carboxy, Sulfoxy, Acyloxyalkyl, oder die Gruppe $-\underset{O}{\underset{\|}{C}}-\underset{H}{N}-R^4$, steht, wobei $R^4$ für Alkyl (geradkettig oder verzweigt), Aryl, Heteroaryl, Aralkyl, Heteroaralkyl oder die Gruppen 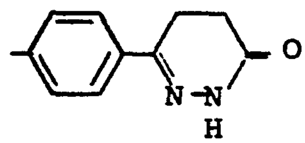 ,

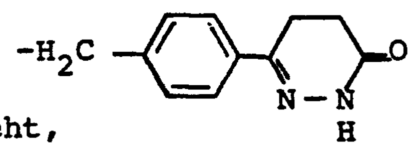

steht,

X Sauerstoff, Schwefel oder NH bedeutet und

n die Werte 1 bis 6 annimmt,

sowie deren physiologisch unbedenklichen Salze wie beispielsweise: Hydrochloride, Hydrogensulfate, Sulfate, Hydrogenphosphate, Formiate, Acetate, Succinate, Maleate, Tartrate, Lactate, Citrate, Fumarate oder Benzoate.

Bevorzugte Verbindungen der Formel (I) sind solche, in denen

$R^1$ für Wasserstoff oder Alkyl (geradkettig oder verzweigt) mit 1 bis 5 C-Atomen, gegebenenfalls substituiert durch Carboxy oder Alkoxycarbonyl (mit 1 bis 4 C-Atomen) steht,

$R^2$ Wasserstoff oder Alkyl (geradkettig oder verzweigt) mit 1 bis 5 C-Atomen bedeutet,

$R^3$ Wasserstoff, Alkyl (geradkettig oder verzweigt) mit 1 bis 6 C-Atomen, Carboxy, Alkoxycarbonyl (mit 1 bis 4 C-Atomen) oder Aryl (mit 6 oder 10 C-Atomen) gegebenenfalls substituiert durch Hydroxyalkyl, Acetoxyalkyl, Benzoyloxyalkyl mit jeweils bis zu 2 C-Atomen, Carboxy, Sulfoxy oder die Gruppe $-\underset{\overset{\|}{O}}{C}-\underset{H}{N}-R^4$ darstellt, wobei

$R^4$ für Alkyl (geradkettig oder verzweigt) mit 1 bis 4 C-Atomen, Phenyl, Benzyl, Pyridyl, Pyridylmethyl, Furfuryl oder die Gruppen

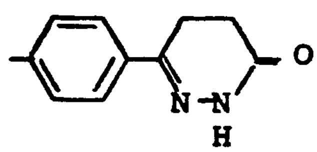 , 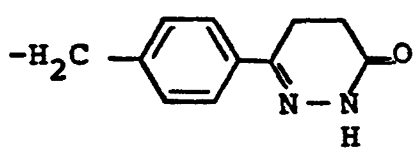

steht,

X Sauerstoff, Schwefel oder NH bedeutet und

n die Werte 1 bis 6 annimmt,

sowie deren physiologisch unbedenklichen Salze.

Besonders bevorzugte Verbindungen der Formel (I) sind diejenigen, in denen

$R^1$ für Wasserstoff, Alkyl (geradkettig oder verzweigt) mit 1 bis 4 C-Atomen steht,

$R^2$ für Alkyl (geradkettig oder verzweigt) mit 1 bis 5 C-Atomen steht,

$R^3$ für Wasserstoff, Alkyl (geradkettig oder verzweigt) mit 1 bis 6 C-Atomen, Carboxy, Alkoxycarbonyl (mit 1 bis 4 C-Atomen), oder Aryl (mit 6 oder 10 C-Atomen), gegebenenfalls substituiert durch Hydroxymethyl, Acetoxymethyl, Carboxy, Sulfoxy oder die Gruppe $-\underset{\overset{\|}{O}}{C}-\underset{H}{N}-R^4$, steht, wobei

$R^4$ für Alkyl (geradkettig oder verzweigt) mit 1 bis 4 C-Atomen, Phenyl, Benzyl, Pyridyl, Pyridylmethyl, Furfuryl oder die Gruppen

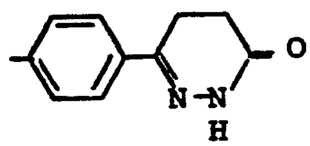 , 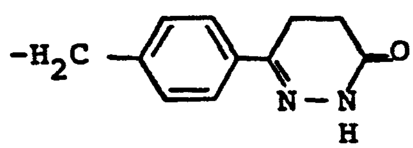

steht,

X Sauerstoff oder NH darstellt, und

n die Werte 1 bis 6 annimmt,

sowie deren physiologisch unbedenklichen Salze.

(A) Die erfindungsgemäßen Verbindungen der Formel (I), worin $R^3$ jedoch nicht für einen Arylrest, der durch die Gruppe $-\underset{\underset{O}{\|}}{C}-\underset{H}{N}-R^4$ substituiert ist, stehen darf,

sind erhältlich, indem man Ketoverbindungen der allgemeinen Formel (II)

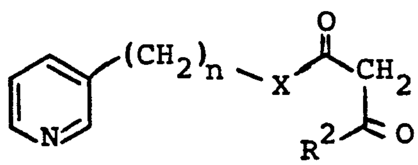

(II)

in welchen

$R^2$, X und n die oben angegebene Bedeutung haben,

mit Aldehyden der allgemeinen Formel (III)

$$R^3-\underset{\underset{O}{\|}}{C}-H \qquad \text{(III)}$$

in welcher

$R^3$ die oben angegebene Bedeutung hat, jedoch nicht für einen durch die Gruppe $-\underset{\underset{O}{\|}}{C}-\underset{H}{N}-R^4$ substituierten Arylrest stehen darf,

und Aminen der allgemeinen Formel (IV),

$$R^1 - NH_2 \qquad \text{(IV)}$$

in welcher

$R^1$ die oben angegebene Bedeutung hat,

in Gegenwart eines inerten organischen Lösungsmittels umsetzt und gegebenenfalls in ihre physiologisch unbedenklichen Salze überführt.

Verwendet man Acetessigsäure-3-(3-pyridyl)propylester, 4-Formylbenzolsulfonsäure und Ammoniak als Ausgangsstoffe, so kann der Reaktionsablauf durch folgendes Formelschema wiedergegeben werden:

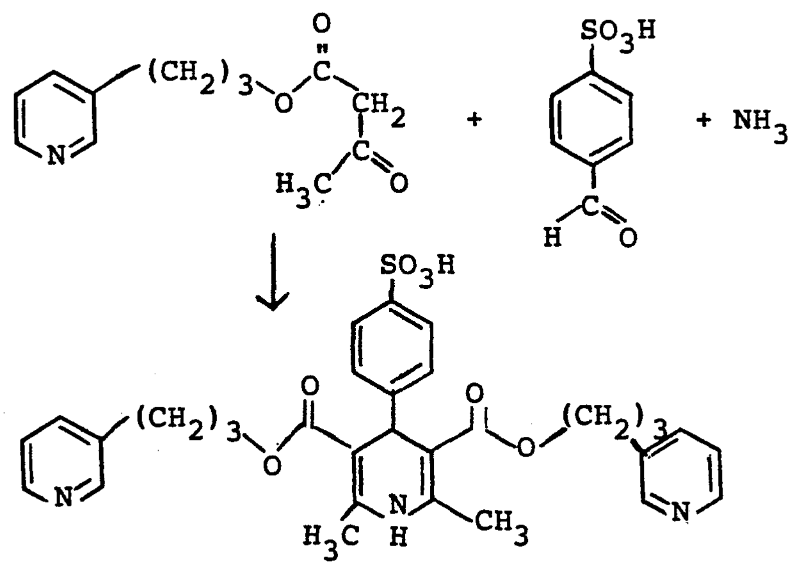

Bei der Durchführung des erfindungsgemäßen Verfahrens ist das Molverhältnis der Ketoverbindungen (II) zu den Aldehyden (III) zwischen 10:1 und 0,1:1, bevorzugt zwischen 2:1 und 0,5:1. Das verwendete Amin (IV) wird zweckmäßig im Überschuß zugegeben (etwa 1-3 molare Menge bezogen auf 1 Mol Aldehyd).

Als Lösungsmittel kommen Wasser und alle inerten organischen Lösungsmittel in Frage. Dazu gehören vorzugsweise Alkohole wie Methanol, Ethanol, Propanol, Isopropanol oder Ether wie Diethylether, Tetrahydrofuran oder Dioxan, Eisessig, Dimethylformamid, Dimethylsulfoxid, Acetonitril, Pyridin oder Hexamethylphosphorsäuretriamid.

Die Reaktionstemperaturen können in einem größeren Bereich variiert werden. Im allgemeinen arbeitet man zwischen 10 und 200°C, insbesondere zwischen 20 und 150°C.

Die Umsetzung kann bei Normaldruck, aber auch bei erhöhtem Druck durchgeführt werden. Im allgemeinen arbeitet man bei Normaldruck.

(B) Die erfindungsgemäßen Verbindungen der Formel (I) worin

$R^1$,$R^2$,X und n die oben angegebene Bedeutung haben,

$R^3$ jedoch einen durch die Gruppe $-\underset{\overset{\|}{O}}{C}-\underset{H}{N}-R^4$ substituierten Arylrest darstellt, wobei $R^4$ wiederum die oben angegebene Bedeutung hat,

können wie folgt hergestellt werden:

Verbindungen der allgemeinen Formel (I) in welcher

$R^1$, $R^2$, X und n die oben angegebene Bedeutung haben,

$R^3$ jedoch für -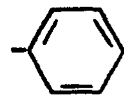-COOH steht,

werden zunächst in aktivierte Carbonsäurederivate der allgemeinen Formel (V)

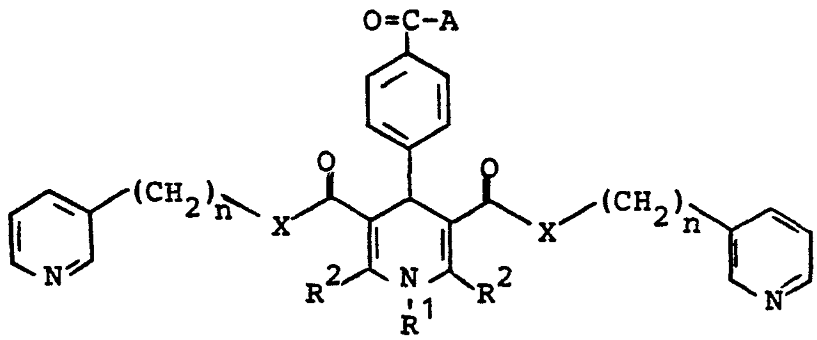 (V)

in welcher

$R_1$, $R_2$, X und n die oben angegebene Bedeutung haben und

A für einen elektronenziehenden Rest, wie er in der Peptidchemie zur Aktivierung von Carbonsäuren üblich ist, steht,

übergeführt und diese anschließend mit Aminen der allgemeinen Formel (VI)

$$H_2N-R^4 \qquad (VI)$$

in welcher

$R^4$ die oben angegebene Bedeutung hat,

in Gegenwart von Basen gegebenenfalls in inerten organischen Lösungsmitteln umgesetzt.

Gegebenenfalls werden sie in ihre physiologisch unbedenklichen Salze überführt.

Verwendet man 4-(4-Carboxyphenyl)-1,4-dihydro-2,6-dimethyl-pyridin-3,5-dicarbonsäure-di-3-(3-pyridyl)-propylester und Benzylamin als Ausgangsstoffe, kann man den Reaktionsverlauf durch folgendes Schema darstellen:

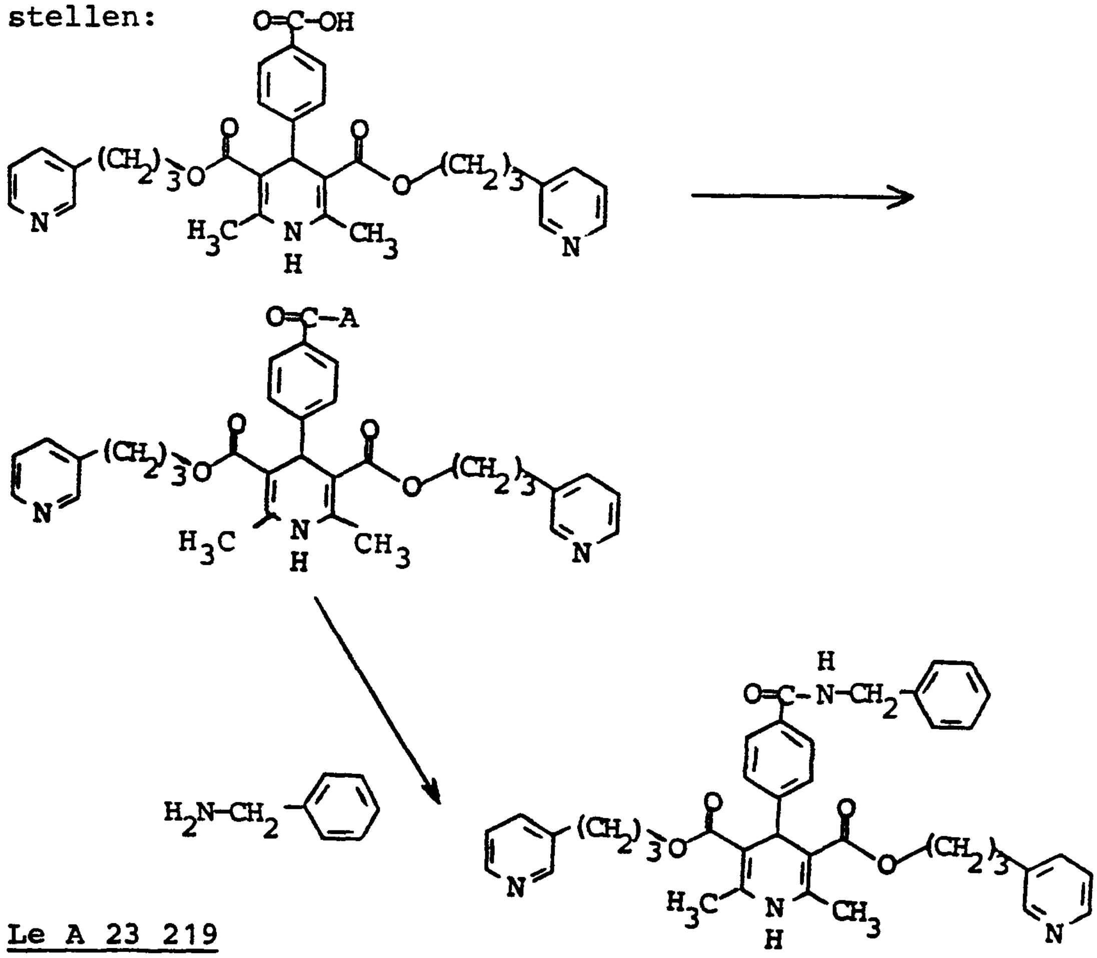

Bei der Durchführung des erfindungsgemäßen Verfahrens kann das Mol-Verhältnis der Amine (VI) zu den aktiven Carbonsäurederivaten (V) im Bereich von 10:1 bis 0,1:1 variieren. Vorzugsweise arbeitet man im Bereich zwischen 2:1 und 0,5:1.

Die Umsetzung kann in Gegenwart der üblichen Basen wie Triethylamin, Ethyldiisopropylamin, N-Methylmorpholin, Pyridin, 4-Diethylaminopyridin oder N,N-Dimethylanilin durchgeführt werden.

Als Lösungsmittel kommen die üblichen inerten organischen Lösungsmittel in Frage. Hierzu gehören bevorzugt chlorierte Kohlenwasserstoffe wie Dichlormethan, Trichlormethan oder 1,2-Dichlorethan, Ether wie Diethylether, Tetrahydrofuran, Dioxan oder 1,2-Dimethoxyethan, aromatische Kohlenwasserstoffe wie Benzol oder Toluol, Acetonitril, Nitromethan, Dimethylformamid, Hexamethylphosphorsäuretriamid, Pyridin, Essigsäureethylester, Aceton.

Die Reaktionstemperaturen liegen im allgemeinen zwischen -70°C und +60°C, bevorzugt zwischen -60°C und +20°C.

Die Umsetzung kann bei Normaldruck, aber auch bei erhöhtem Druck durchgeführt werden. Im allgemeinen arbeitet man bei Normaldruck.

Die Ketoverbindungen der Formel (II) sind zum Teil bekannt oder können nach an sich bekannten Methoden hergestellt werden (vgl. D. Borrmann, "Umsetzungen von Diketen mit Alkoholen, Phenolen und Mercaptanen" in Houben-Weyl, Methoden der organischen Chemie, Band VII/4, 230 ff (1968)).

Die Aldehyde der Formel (III) sind zum Teil bekannt oder können in angegebener Weise nach bekannten Methoden hergestellt werden.

Die Amine der allgemeinen Formeln (IV) und (VI) sind bekannt.

Die Verbindungen der allgemeinen Formel (V) gehören - je nach der Bedeutung des Restes A - folgenden Substanzklassen an und können nach bekannten Verfahren hergestellt werden (vgl. Houben-Weyl, Methoden der organischen Chemie, Band XV/2, 1974): Ethoxycarbonyl- und Diethoxycarbonyl-methylester, 2-Oxopropylester, 2-Diethylaminoethylester, Brommethylester, Cyanmethylester, Aminocarbonyl-methylester, Propargylester, Glykolsäureester, Ribosylester, Phenylester, Nitrophenylester, Dinitrophenylester, Dichlor-nitrophenylester, Trichlorphenylester, Pentachlorphenylester, Pentafluorphenylester, 4-Methylsulfonylphenylester, Phenylazophenylester, 4-Cyanphenylester, Chinolyl-(8)-ester, 1-Ethoxycarbonyl-2-ethoxy-1,2-dihydro-chinolylester, Pyridyl-(3)-ester, 2-Hydroxyphenylester, 4-Nitro-guaiacylester, 4-Dimethylaminophenylester, 4-Aminosulfonylphenylester, 4-(Acetylamino-sulfonyl)-phenylester, 4-Propionyl-phenylester, Vinylester, 1-Methyl-2-acetyl-

vinylester, 2,2-Diphenylvinylester, 2-Cyan-2-phenylvinylester, Methoxy-methylester, Tetrahydropyranyl-(2)-ester, 1-Methoxy-vinylester, 1-Ethoxy-vinylester, Dimethylaminovinylester, N,N'-Dicyclohexyllactimester, N-Ethyl-N'-(3-dimethylaminopropyl)-lactimester, 2-Hydroxypyridylester, O-Acyl-N,N-dimethyl-hydroxylamine, O-Acyl-N,N-diethyl-hydroxylamine, O-Acyl-N,N-dibenzyl-hydroxylamine, (N-Hydroxy-piperidin)-ester, O-Acyl-N-isopropyliden-hydroxylamine, (N-Hydroxy-pivaloamid)-ester, (N-Hydroxy-benzamid)-ester, [1,2-Dihydro-pyridonyl-(1)]-ester, (N-Hydroxy-succinimid)-ester, (N-Hydroxy-glutarimid)-ester, (N-Hydroxy-phthalimid)-ester, (N-Hydroxy-chinolinsäureimid)-ester, O-Methyl-Kohlensäureanhydride, O-Ethyl-Kohlensäureanhydride, O-Isobutyl-Kohlensäureanhydride, O-Benzyl-Kohlensäureanhydride, O-Phenyl-Kohlensäureanhydride, 2-Ethylbuttersäureanhydride, 2,2-Dimethyl-propionsäureanhydride, Diphenylessigsäureanhydride, Benzoesäureanhydride, 4-Methoxybenzoesäureanhydride, O,O-Dibenzylphosphorsäureanhydride, O,O-Di-(4-nitrobenzyl)-phosphorsäureanhydride, Methansulfonsäureanhydride, Benzolsulfonsäureanhydride, 4-Methylbenzolsulfonsäureanhydride, 4-Nitrobenzolsulfonsäureanhydride, 4-Methoxybenzolsulfonsäureanhydride, Trifluormethylsulfonsäureanhydride, Nonafluorbutylsulfonsäureanhydride, Phenylthioester, 4-Nitrophenylthioester, Phenylselenoester, Carbonsäureazide, Carbonsäureimidazolide, Carbonsäure-1,2,4-triazolide, Carbonsäure-1,2,4-oxadiazolinone-(5), Carbonsäurechloride, Carbonsäurebromide, Carbonsäureiodide, Carbonsäurecyanide

Die vorstehenden Herstellungsverfahren sind lediglich zur Verdeutlichung angegeben und die Herstellung der Verbindung (I) ist nicht auf dieses Verfahren beschränkt, sondern jede Modifikation der Verfahren ist in üblicher Weise für die Herstellung der erfindungsgemäßen Verbindungen (I) anwendbar.

Die vorliegende Erfindung betrifft auch die Verwendung der erfindungsgemäßen Stoffe der Formel (I) als Hemmer/Stimulatoren von enzymatischen Reaktionen im Rahmen des Arachidonsäurestoffwechsels. Derartige Substanzen sind zur Verhütung und Behandlung von Erkrankungen der Atemwege wie Emphysem, Schocklunge, pulmonaler Hypertonie, Ödem, Thrombose und Thromboembolie, Ischämien (periphere, coronare, cerebrale Durchblutungsstörungen), Herz- und Kirninfarkte, Herzrhythmusstörungen, Angina pectoris, Hypertonie sowie Arteriosklerose geeignet.
Die erfindungsgemäßen Stoffe sind bevorzugt Hemmer der Thromboxansynthese und stimulieren gleichzeitig die Prostacyclinsynthese.

Die neuen Wirkstoffe können in bekannter Weise in die üblichen Formulierungen (wie z.B. Tabletten, Kapseln, Dragees, Pillen, Granulate, Cremes, Suppositorien, Emulsionen, Suspensionen sowie Infusions- und Injektionslösungen) unter Verwendung inerter, nichttoxischer, pharmazeutisch geeigneter Trägerstoffe oder Lösungsmittel übergeführt werden.

Besonders geeignet sind Formulierungen, die etwa 0,1 bis 10 Gew.-% an Wirkstoff enthalten, vorzugsweise wäßrige Lösungen. Besonders bevorzugt sind wäßrige Lösungen mit einem pH-Wert zwischen 6 und 8.

Die Formulierungen erfolgen nach den üblichen Methoden, beispielsweise durch Verstrecken der Wirkstoffe mit Lösungsmitteln und/oder Trägerstoffen, gegebenenfalls unter Verwendung von Emulgiermitteln und/oder Dispergiermitteln wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel wie Paraffine (z.B. Erdölfraktionen), pflanzliche Öle (z.B. Erdnuß-/ Sesamöl), Alkohole (z.B. Ethylalkohol, Glycerin), Glykole (z.B. Propylenglykol, Polyethylenglykol), feste Trägerstoffe wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-, Traubenzucker), Emulgiermittel (z.B. Polyoxyethylen-Fettsäureester, Polyoxyethylen-Fettalkoholether, Alkylsulfonate, Arylsulfonate), Dispergiermittel (z.B. Lignin, Sulfitablaugen, Methylcellulose, Stärke, Polyvinylpyrrolidon) und Gleitmittel (z.B. Magnesiumstearat, Talkum, Stearinsäure, Natriumlaurylsulfat).

Die Applikation erfolgt in üblicher Weise je nach Bedarf lokal, oral oder parenteral.

Die Dosierung erfolgt im allgemeinen in einem Bereich von 0,05 bis 100 mg/kg Körpergewicht, insbesondere von 0,1 bis 50 mg/kg Körpergewicht.

Im Unterschied zu Indometacin, welches die Prostaglandinsynthese aus der Arachidonsäure insgesamt hemmt, greifen die erfindungsgemäß einzusetzenden Wirkstoffe viel spezifischer in den Stoffwechsel der für die Bildung des Prostacyclins ($PGI_2$) und des Thromboxans maßgebende Enzyme ein, so daß nicht nur der schädigende, gefäßverengende und arrythmienerhöhende Einfluß des Thromboxans reduziert, sondern auch der gefäßerweiternde Einfluß des $PGI_2$'s durch Stimulierung seiner Bildung erhöht wird.

Die biologische Wirkung der erfindungsgemäß hergestellten Verbindung wurde durch folgende Experimente nachgewiesen:

I. $^3$H-Arachidonsäuremetabolismus

Der Arachidonsäuremetabolismus in Human-Thrombozyten wurde mit Hilfe von Tritium-markierter Arachidonsäure untersucht. Die Thrombozyten metabolisieren die Arachidonsäure über den Cyclooxygenaseweg zu $TXA_2$ und HHT und über den Lipogenaseweg zu 12-HETE, die dünnschichtchromatographisch getrennt werden können (vgl. Bailey, J.M. et al., Prostaglandins 13, 479-492, 1977). Inhibitoren der einzelnen enzymatischen Reaktionen verändern das chromatographische Verteilungsmuster in charakteristischer Weise.

Gewaschene Human-Thrombozyten von gesunden Spendern, die 14 Tage kein Medikament eingenommen hatten, wurden mit Prüfsubstanz 2 min bei 37°C inkubiert und anschließend mit $^3$H-Arachidonsäure weitere 10 min bei 37°C inkubiert. Die Suspension wurde angesäuert und mit Essigester extrahiert. Der Essigester wurde unter Stickstoffatmosphäre abgedampft und der Rückstand in Methanol/Trichlormethan (1:1) aufgenommen und auf DC-Plastikfolien aufgetragen. Die Trennung erfolgte mit einem Fließmittelgemisch Trichlormethan/Methanol/Eisessig/Wasser (80:8:1:0,8). Die Verteilung der Radioaktivität wurde mittels eines Radioscanners gemessen.

II. Prostacyclinstimulation

Außerdem stimulieren die erfindungsgemäß zu verwendenden 1,4-Dihydropyridine die Synthese von $PGI_2$. $PGI_2$ wirkt im Gegensatz zu dem vasokonstriktorischen und thrombozytenaggregationsauslösenden Thromboxan gefäßdilatierend und thrombozytenaggregationshemmend.

Stimulation im Vollblut

Im Vollblut läßt sich durch Kollagen die Bildung von $PGI_2$ induzieren. Die in Thrombozyten gebildeten Endoperoxide werden wahrscheinlich durch Leukozyten-Lipoxygenase in $PGI_2$ umgewandelt. Das

stabile Endprodukt der $PGI_2$-Umwandlung, 6-Keto-$PGF_{1\alpha}$, wird radioimmunologisch bestimmt.

## III. Thrombozytenaggregation

Thrombozyten und deren Adhäsion - sowie Aggregationsfähigkeit - sind, besonders im arteriellen Schenkel des Gefäßsystems, ein wesentlicher pathogenetischer Faktor bei der Entstehung von Thrombosen. Überraschenderweise waren die erfindungsgemäßen Verbindungen auch in diesem Test wirksam, was die Substanz in Verbindung mit den anderen Eigenschaften als besonders günstig erscheinen läßt.

Für die in vitro Versuche wurde Blut von gesunden Probanden beiderlei Geschlechts verwendet. Als Antikoagulans wurde ein Teil 3,8 %ige wäßrige Natriumzitratlösung 9 Teilen Blut zugemischt. Mittels Zentrifugation erhält man aus diesem Blut plättchenreiches Zitratplasma (PRP) (vgl. Jürgens/Beller "Klinische Methoden der Blutgerinnungsanalyse"; Thieme Verlag, Stuttgart 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (vgl. Born, B.V.R., J. Physiol. (London), 162, 67 (1962)) im Aggregometer bei 37°C bestimmt (vgl. Therapeutische Berichte 47, 80-86 (1975)).

Hierzu wurde die vorinkubierte Probe mit 0,1 ml Kollagen, einem aggregationsauslösenden Agens, versetzt.
Die Veränderung der optischen Dichte in der Probe des PRP wurde während einer Zeitdauer von 6 min aufgezeichnet und der Ausschlag nach 6 min bestimmt. Hieraus wird die prozentuale Hemmung gegenüber der Kontrolle berechnet. Als Grenzkonzentration wird der Bereich der minimal effektiven Konzentration angegeben.

Tabelle 1

Hemmung der $TXA_2$-Synthese

| Bsp. Nr. | Grenzkonzentration für Hemmung (mg/l) |
|---|---|
| 1 | 0,3 - 0,1 |
| 2 | 1 - 0,3 |
| 4 | 1 - 0,3 |
| 5 | 3 - 1 |
| 6 | 10 - 3 |
| 7 | 10 - 3 |
| 8 | 0,1 - 0,03 |
| 9 | 0,3 - 0,1 |
| 10 | 1 - 0,3 |
| 11 | 1 - 0,3 |

Tabelle 2

Prostacyclinstimulation

| Bsp. Nr. | Konzentration (mg/l) | Stimulation (%) |
|---|---|---|
| 1 | 10 - 3 | 158 |
| 4 | 0,3 - 0,1 | ~50 |
| 5 | 0,3 - 0,1 | 100 |
| 9 | 1 - 0,1 | |
| 10 | 0,1 - 0,03 | ~50 |
| 11 | 0,1 - 0,03 | ~100 |

Tabelle 3

Hemmung der Thrombozytenaggregation

| Bsp. Nr. | Grenzkonzentration für Hemmung (mg/l) |
|---|---|
| 8 | 3 - 1 |

## Herstellungsbeispiele

### Beispiel 1

**1,4-Dihydro-4-(4-hydroxymethylphenyl)-2,6-dimethyl-pyridin-3,5-dicarbonsäure-di-3-(3-pyridyl)propylester**

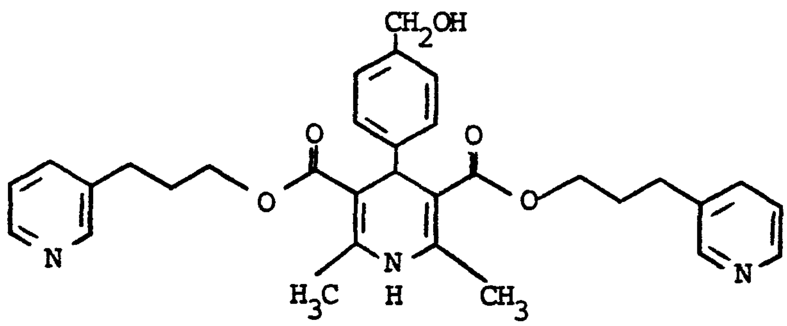

a) 4-Hydroxymethylbenzaldehyd

200 g (1,44 mol) Terephthaldialdehyd werden in 2000 ml Methanol suspendiert und unter Eiskühlung mit 23,5 g (0,62 mol) Natriumborhydrid versetzt. Nach Erwärmen auf Raumtemperatur wird im Vakuum auf ein Viertel des Volumens eingeengt und mit Dichlormethan 3 mal extrahiert. Nach Trocknen mit Natriumsulfat und Abziehen des Dichlormethans kristallisierte aus Ether 4-Hydroxymethylbenzylalkohol aus. Nach Eindampfen des Filtrats erhielt man 61,7 g kristallines Produkt.
Ausbeute: 61,7 g (34,5 % der Theorie)
Schmelzpunkt: 48°C

b) 1,4-Dihydro-4-(4-hydroxymethylphenyl)-2,6-dimethylpyridin-3,5-dicarbonsäure-di-3-(3-pyridyl)propylester

5 g Acetessigsäure-3-(3-pyridyl)propylester werden zusammen mit 0,85 ml 25 % wäßriger Ammoniaklösung und 1,54 g 4-Hydroxymethylbenzaldehyd in 20 ml Isopropanol 48 h unter Rückfluß erhitzt. Nach dem Eindampfen der Reaktionslösung kristallisiert das Produkt aus Ether und wird aus Essigester umkristallisiert.
Ausbeute: 0,7 g (12 % der Theorie)
Schmelzpunkt: 95 bis 100°C

Beispiel 2

4-(4-Acetoxymethylphenyl)-1,4-dihydro-2,6-dimethylpyridin-3,5-dicarbonsäure-di-3-(3-pyridyl)propylester

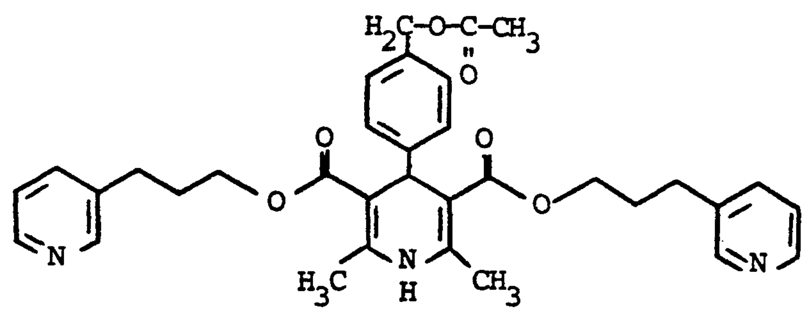

a) 4-Acetoxymethylbenzaldehyd

54,2 g 4-Hydroxymethylbenzaldehyd werden zusammen mit 40 g Triethylamin in 350 ml Dichlormethan gelöst. Nach Zutropfen von 31,2 g Acetylchlorid bei

0 bis 5°C läßt man die Reaktionsmischung über Nacht bei Raumtemperatur stehen. Anschließend wird das ausgefallene Triethylammoniumchlorid abgesaugt und das Filtrat 3 mal mit gesättigter Bicarbonatlösung extrahiert. Nach Trocknen mit Natriumsulfat wird der Rückstand im Hochvakuum destilliert. Man erhält so 47,9 g farblosen Feststoff.
Ausbeute: 47,9 g (85 % der Theorie)
Schmelzpunkt: 32°C.

b) 4-(4-Acetoxymethylphenyl)-1,4-dihydro-2,6-dimethyl-pyridin-3,5-dicarbonsäure-di-3-(3-pyridyl)propylester

5 g Acetessigsäure-3-(3-pyridyl)propylester werden zusammen mit 0,85 ml 25 % wäßriger Ammoniaklösung und 2,01 g 4-Acetoxymethylbenzaldehyd in 20 ml Isopropanol 24 h unter Rückfluß erhitzt. Die Reaktionslösung wird eingedampft und der Rückstand an Kieselgel mit einem Gemisch aus Dichlormethan und Methanol im Verhältnis 95:5 als Elutionsmittel chromatographiert. Man erhält so eine Fraktion, die nach dem Eindampfen das Produkt als Feststoff ergibt.
Ausbeute: 1,3 g (20 % der Theorie)
Schmelzpunkt: 89 bis 94°C.

Beispiel 3

1,4-Dihydro-2,6-dimethyl-4-(4-sulfoxyphenyl)-pyridin-3,5-dicarbonsäure-di-3-(3-pyridyl)propylester

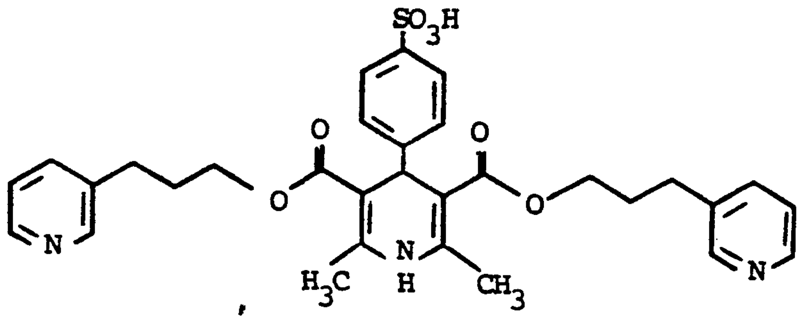

15 g Acetessigsäure-3-(3-pyridyl)propylester werden zusammen mit 2,55 ml 25 % wäßriger Ammoniaklösung und 6,3 g 4-Formylbenzolsulfonsäure in 60 ml Isopropanol 24 h unter Rückfluß erhitzt. Die Reaktionsmischung wird filtriert und eingedampft. Der Rückstand kristallisiert aus einem Gemisch von Dichlormethan mit wenig Methanol.

Ausbeute: 9,6 g (48 % der Theorie)

Schmelzpunkt: 130°C (Zers.).

Beispiel 4

1,4-Dihydro-2,6-dimethyl-pyridin-3,5-dicarbonsäure-di-3-(3-pyridyl)propylester

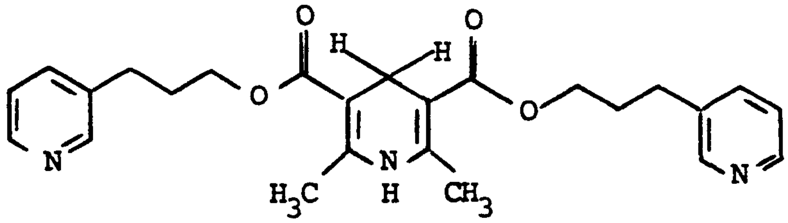

5 g Acetessigsäure-3-(3-pyridyl)propylester werden zusammen mit 0,85 ml 25 % wäßriger Ammoniaklösung und 0,89 ml 35 % wäßriger Formaldehydlösung in 20 ml Isopropanol 4 h unter Rückfluß erhitzt. Anschließend wird im Vakuum eingedampft. Der Rückstand kristallisiert aus Ether.
Ausbeute: 1,4 g (28 % der Theorie)
Schmelzpunkt: 130°C

Beispiel 5

1,4-Dihydro-2,6-dimethyl-pyridin-3,4,5-tricarbonsäure-4-methylester-3,5-di-3-(3-pyridyl)propylester

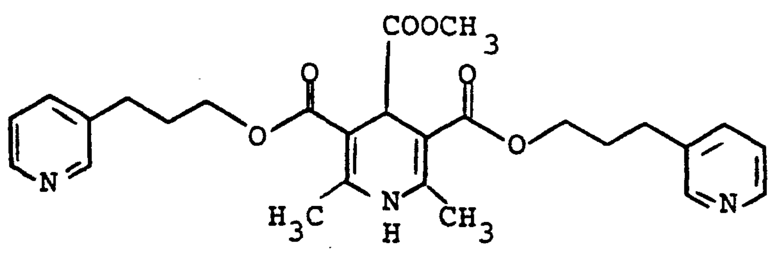

25,1 g Acetessigsäure-3-(3-pyridyl)propylester werden zusammen mit 5 g Glyoxalsäuremethylester und 4,24 ml 25 % wäßriger Ammoniaklösung in 100 ml Isopropanol 1,5 h unter Rückfluß erhitzt. Die Reaktionslösung wird eingedampft, der Rückstand in Essigester aufgenommen und 3 mal mit 2 molarer Natronlauge gewaschen. Die organische Phase wird mit Natriumsulfat getrocknet und eingedampft. Der Rückstand kristallisiert aus Ether.
Ausbeute: 8 g (28 % der Theorie)
Schmelzpunkt: 118 bis 122°C.

Beispiel 6

4-(4-Carboxyphenyl)-1,4-dihydro-2,6-dimethylpyridin-3,5-dicarbonsäure-di-3-(3-pyridyl)propylester

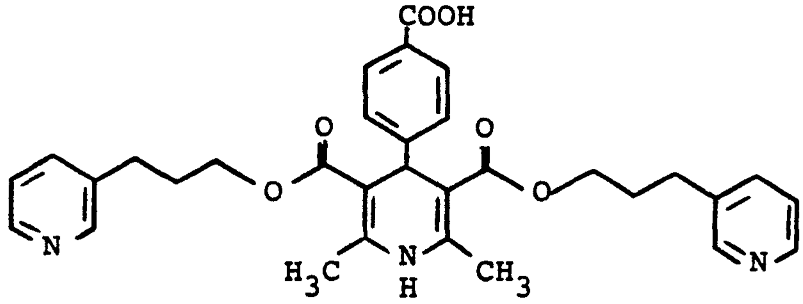

22 g Acetessigsäure-3-(3-pyridyl)propylester werden zusammen mit 7,5 g 4-Formylbenzoesäure und 4 ml 25 % wäßriger Ammoniaklösung in 80 ml Isopropanol 2 h unter Rückfluß erhitzt. Die Reaktionslösung wird eingedampft, der Rückstand kristallisiert aus einem Gemisch von Isopropanol mit Essigester.
Ausbeute: 11,7 g (42 % der Theorie)
Schmelzpunkt: 165 bis 168°C.

Beispiel 7

4-(4-Carboxyphenyl)-1,4-dihydro-2,6-dimethyl-pyridin-3,5-dicarbonsäure-di-(3-pyridyl)-methylester

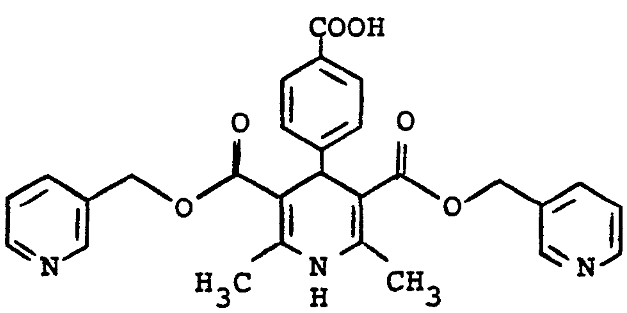

8,6 g Acetessigsäure-3-pyridylmethylester werden zusammen mit 3,75 g 4-Formylbenzoesäure und 2 ml 25 % wäßriger Ammoniaklösung in 40 ml Isopropanol 2 h unter Rückfluß erhitzt. Die Reaktionslösung wird gründlich eingedampft, der Rückstand kristallisiert aus Essigester.
Ausbeute: 8 g (64 % der Theorie)
Schmelzpunkt: 205 bis 207°C.

Beispiel 8

1,4-Dihydro-2,6-dimethyl-4-/4-/N-(4-(2,3,4,5-tetrahydro-3-oxo-pyridazin-6-yl)phenyl)7carbamoylphenyl7-pyridin-3,5-dicarbonsäure-di-3-(3-pyridyl)propylester

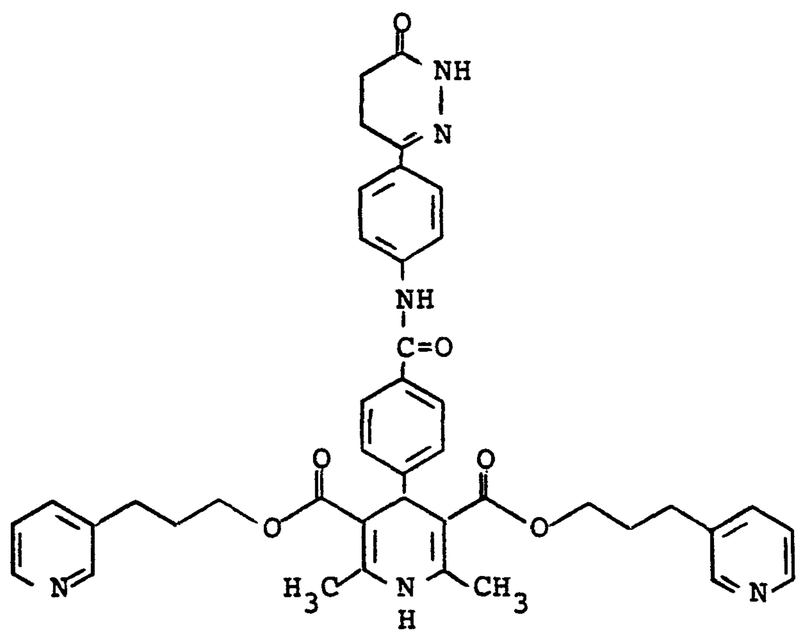

2,2 g 4-(4-Carboxyphenyl)-1,4-dihydro-2,6-dimethyl-pyridin-3,5-dicarbonsäure-di-3-(3-pyridyl)propylester werden in 20 ml trockenem Dimethylformamid gelöst und mit 1 ml Triethylamin versetzt. Nach Abkühlen dieser Lösung auf -60°C werden bei dieser Temperatur 0,31 ml Methansulfonsäurechlorid zugetropft und die Reaktionsmischung 15 min bei -60°C gerührt. Dann werden 0,8 g 6-(4-Aminophenyl)-2,3,4,5-tetrahydro-3-oxo-pyridazin, gelöst in 20 ml trockenem Dimethylformamid, bei -60°C zugetropft. Man läßt die Reaktionsmischung langsam auf Raumtemperatur erwärmen und rührt 3 h bei Raumtemperatur nach. Die Reaktionsmischung wird mit Wasser verdünnt und 3 mal mit Essigester extrahiert. Die vereinigten Essigesterphasen werden einmal mit 1 molarer Natronlauge gewaschen, mit Natriumsulfat getrocknet und eingedampft. Man erhält so 1,4 g festen Schaum.
Ausbeute: 1,4 g (48 % der Theorie)
Schmelzpunkt: 61 bis 66°C.

Beispiel 9

1,4-Dihydro-2,6-dimethyl-4-[4-N-(3-pyridyl)carbamoyl-phenyl]pyridin-3,5-dicarbonsäure-di-3-(3-pyridyl)-propylester

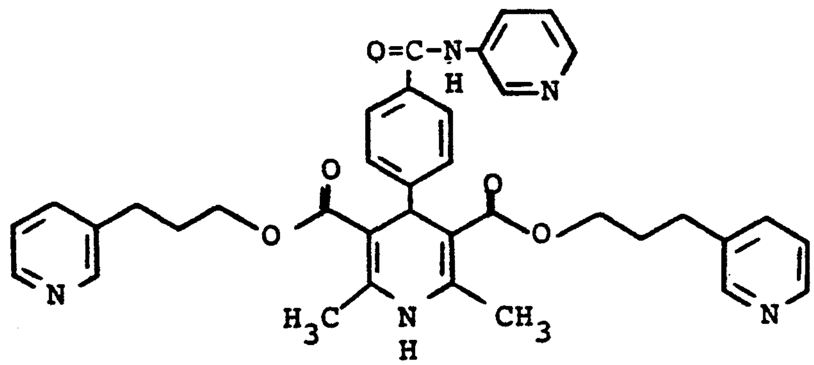

2 g 4-(4-Carboxyphenyl)-1,4-dihydro-2,6-dimethyl-pyridin-3,5-dicarbonsäure-di-3-(3-pyridyl)propylester werden in 15 ml Dimethylformamid gelöst und mit 0,74 g Dicyclohexylcarbodiimid und 0,46 g N-Hydroxysuccinimid versetzt. Nach 1 h Rühren bei 0°C werden 0,34 g 3-Aminopyridin zugegeben und die Reaktionsmischung 3 d bei Raumtemperatur gerührt. Anschließend wird noch 3 h bei 40°C gerührt, die Reaktionsmischung filtriert und mit Essigester verdünnt. Die Essigesterlösung wird 3 mal mit Wasser gewaschen, die organische Phase mit Magnesiumsulfat getrocknet und eingedampft. Der Rückstand wird an Kieselgel mit einer Mischung aus Dichlormethan und Methanol im Verhältnis 95:5 als Elutionsmittel chromatographiert. Man erhält so eine Fraktion, die nach dem Eindampfen 1,24 g festes Produkt ergibt.

Ausbeute: 1,24 g (54 % der Theorie)

Schmelzpunkt: °C

Beispiel 10

1,4-Dihydro-2,6-dimethyl-4-/4-N-(3-pyridylmethyl)carbamoyl-phenyl/-pyridin-3,5-dicarbonsäure-di-3-(3-pyridyl)propyl-ester

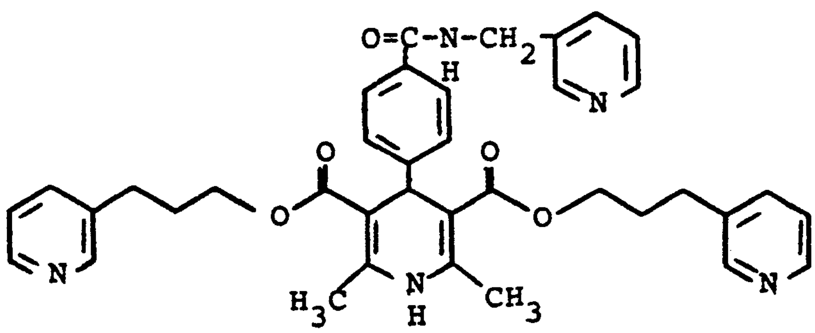

2 g 4-(4-Carboxyphenyl)-1,4-dihydro-2,6-dimethylpyridin-3,5-dicarbonsäure-di-3-(3-pyridyl)propylester werden zusammen mit 0,74 g Dicyclohexylcarbodiimid in 15 ml Dimethylformamid vorgelegt und auf 5°C gekühlt. Nach 15 min Rühren bei dieser Temperatur werden 0,39 g 3-Picolylamin zugegeben und die Reaktionsmischung 1 h bei 5°C gerührt. Anschließend läßt man die Reaktionsmischung 3 d bei Raumtemperatur stehen, saugt vom ausgefallenen Harnstoff ab und dampft das Filtrat ein. Der Rückstand wird an Kieselgel mit einer Mischung aus Dichlormethan und Methanol im Verhältnis 95:5 chromatographiert. Man erhält so eine Fraktion, die nach dem Eindampfen 0,8 g festes Produkt ergibt.

Ausbeute: 0,8 g (34 % der Theorie)

Schmelzpunkt: 57 bis 63°C.

Beispiel 11

4-/4-N-(2-Furylmethyl)carbamoylphenyl/-1,4-dihydro-2,6-dimethyl-pyridin-3,5-dicarbonsäure-di-3-(3-pyridyl)propylester

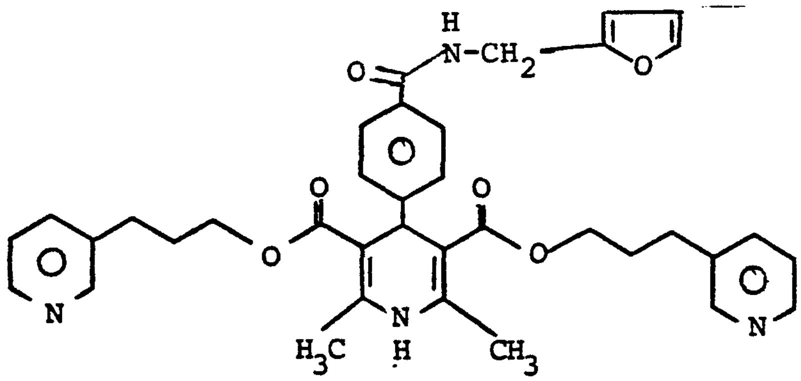

2 g 4-(4-Carboxyphenyl)-1,4-dihydro-2,6-dimethylpyridin-3,5-dicarbonsäure-di-3-(3-pyridyl)propylester werden in 15 ml Dimethylformamid gelöst und bei 0 bis 5°C mit 0,74 g Dicyclohexylcarbodiimid und 0,46 g N-Hydroxysuccinimid versetzt. Nach 1 h Rühren bei 0 bis 5°C werden 0,35 g Furfurylamin zugegeben und die Reaktionsmischung 3 h bei Raumtemperatur gerührt. Anschließend wird die Reaktionsmischung filtriert und mit Essigester verdünnt. Die Essigesterlösung wird 3 mal mit Wasser gewaschen, die organische Phase mit Magnesiumsulfat getrocknet und eingedampft. Der Rückstand wird an Kieselgel mit einer Mischung aus Toluol und Aceton im Verhältnis 1 : 1 als Elutionsmittel chromatographiert. Man erhält so eine Fraktion, die nach dem Eindampfen 0,98 g festes Produkt ergibt.
Ausbeute: 0.98 g (43 % der Theorie)
Schmelzpunkt: 51 - 58°C

Beispiel 12

1,4-Dihydro-2,6-dimethyl-4-(4-N-phenylcarbamoylphenyl)-pyridin-3,5-dicarbonsäure-di-3-(3-pyridyl)propylester

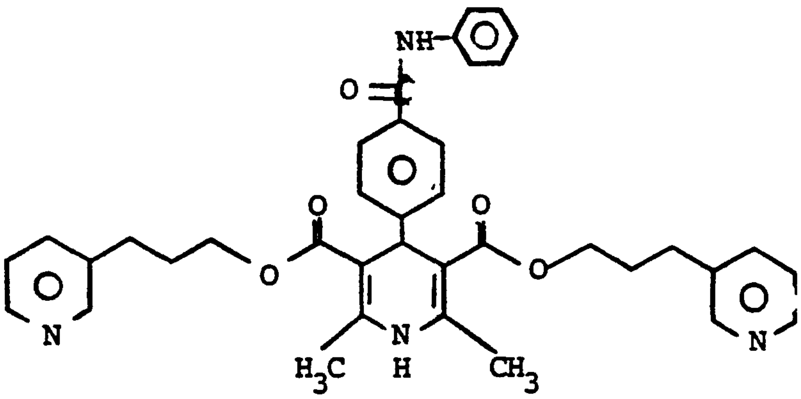

2 g 4-(4-Carboxyphenyl)-1,4-dihydro-2,6-dimethyl-pyridin-3,5-dicarbonsäure-di-3-(3-pyridyl)-propylester werden mit 0,7 g Dicyclohexylcarbodiimid, 0,46 g N-Hydroxysuccinimid und 0,34 g Anilin in zu Beispiel 10 analoger Weise miteinander umgesetzt. Auch die Aufarbeitung und chromatographische Trennung erfolgt analog zu Beispiel 10.

Ausbeute: 1 g (44 % der Theorie)
Schmelzpunkt: 49 - 55°C

Beispiel 13

4-(4-N-Benzylcarbamoylphenyl)-1,4-dihydro-2,6-dimethyl -pyridin-3,5-dicarbonsäure-di-3-(3-pyridyl)propylester

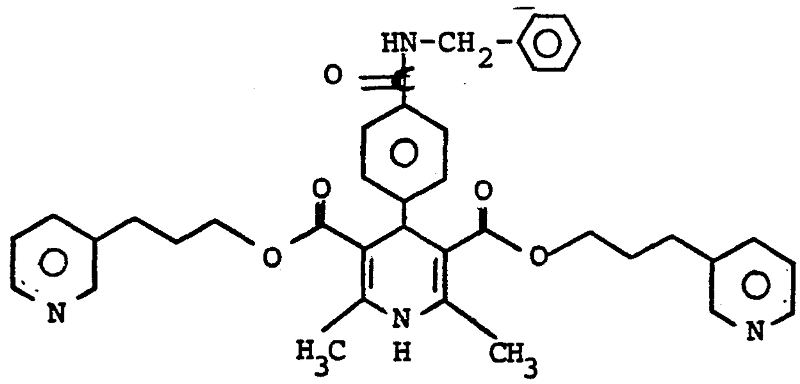

2 g 4-(Carboxyphenyl)-1,4-dihydro-2,6-dimethyl-pyridin-3,5-dicarbonsäure-di-3-(3-pyridyl)propylester werden mit 0,74 g Dicyclohexylcarbodiimid, 0,46 g N-Hydroxysuccinimid und 0,39 g Benzylamin in zu Beispiel 10 analoger Weise miteinander umgesetzt. Auch die Aufarbeitung und die chromatographische Trennung erfolgt analog zu Beispiel 10.

Ausbeute: 1 g (43 % der Theorie)
Schmelzpunkt: 140 - 145°C

Patentansprüche

1) 1,4-Dihydropyridine der allgemeinen Formel (I)

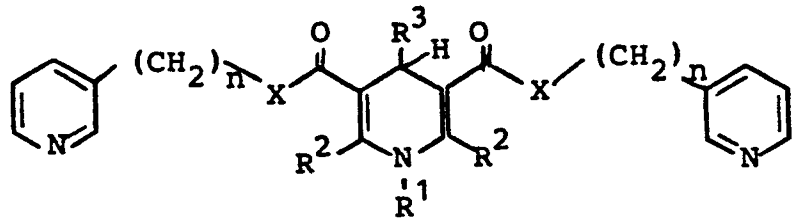

(I)

in der

$R^1$ für Wasserstoff oder Alkyl (geradkettig oder verzweigt), gegebenenfalls substituiert durch Halogen, Nitro, Cyan, Alkoxy, Carboxy, Alkoxycarbonyl oder Carboxamid steht,

$R^2$ für Wasserstoff oder Alkyl (geradkettig oder verzweigt) steht,

$R^3$ für Wasserstoff, Alkyl (geradkettig oder verzweigt), Carboxy, Alkoxycarbonyl oder Aryl, gegebenenfalls substituiert durch: Hydroxyalkyl, Carboxy, Sulfoxy, Acyloxyalkyl oder die Gruppe $-\underset{O}{\overset{}{\underset{\|}{C}}}-\underset{H}{N}-R^4$ steht, wobei $R^4$ für Alkyl (geradkettig oder verzweigt), Aryl, Heteroaryl, Aralkyl, Heteroaralkyl oder die Gruppen 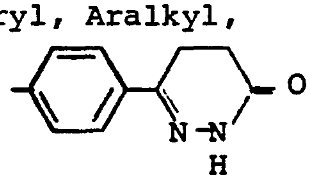 ,

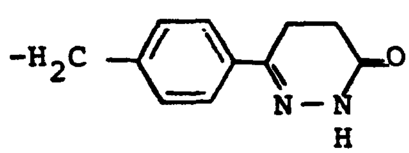

steht,

X Sauerstoff, Schwefel oder NH bedeutet und

n die Werte 1 bis 6 annimmt,

sowie deren physiologisch unbedenklichen Salze.

2) Verbindungen der allgemeinen Formel (I) gemäß Anspruch 1 in der

$R^1$ für Wasserstoff oder Alkyl (geradkettig oder verzweigt) mit 1 bis 5 C-Atomen, gegebenenfalls substituiert durch Carboxy oder Alkoxycarbonyl (mit 1 bis 4 C-Atomen) steht,

$R^2$ Wasserstoff oder Alkyl (geradkettig oder verzweigt) mit 1 bis 5 C-Atomen bedeutet,

$R^3$ Wasserstoff, Alkyl (geradkettig oder verzweigt) mit 1 bis 6 C-Atomen, Carboxy, Alkoxycarbonyl (mit 1 bis 4 C-Atomen) oder Aryl (mit 6 oder 10 C-Atomen) gegebenenfalls substituiert durch Hydroxyalkyl, Acetoxyalkyl, Benzoyloxyalkyl mit jeweils bis zu 2 C-Atomen, Carboxy, Sulfoxy oder die Gruppe $-\underset{\overset{\|}{O}}{C}-\underset{H}{N}-R^4$

darstellt, wobei

$R^4$ Alkyl (geradkettig oder verzweigt) mit 1 bis 4 C-Atomen, Phenyl, Benzyl, Pyridyl, Pyridylmethyl, Furfuryl oder die Gruppen

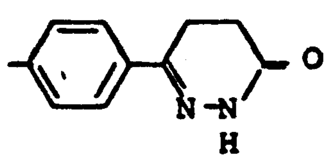 , 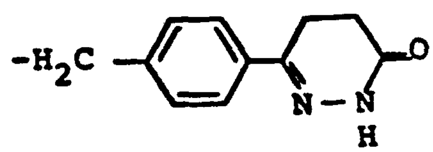

steht,

X Sauerstoff, Schwefel oder NH bedeutet und

n die Werte 1 bis 6 annimmt,

sowie deren physiologisch unbedenklichen Salze.

3) Verbindungen der allgemeinen Formel (I) gemäß Anspruch 1 in der

$R^1$ Wasserstoff, Alkyl (geradkettig oder verzweigt) mit 1 bis 4 C-Atomen,

$R^2$ Alkyl (geradkettig oder verzweigt) mit 1 bis 5 C-Atomen,

$R^3$ Wasserstoff, Alkyl (geradkettig oder verzweigt) mit 1 bis 6 C-Atomen, Carboxy, Alkoxycarbonyl (mit 1 bis 4 C-Atomen), oder Aryl (mit 6 oder 10 C-Atomen), gegebenenfalls substituiert durch Hydroxymethyl, Acetoxymethyl, Carboxy, Sulfoxy oder die Gruppe $-\underset{O}{\overset{\|}{C}}-\underset{H}{N}-R^4$, wobei

$R^4$ für Alkyl (geradkettig oder verzweigt) mit 1 bis 4 C-Atomen, Phenyl, Benzyl, Pyridyl, Pyridylmethyl, Furfuryl oder die Gruppen

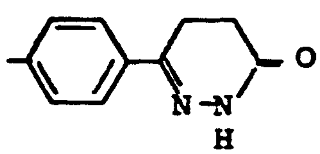 , 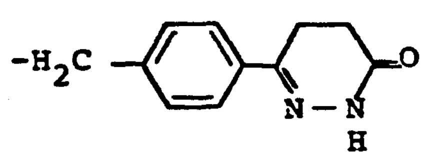

steht,

X Sauerstoff oder NH darstellt, und

n die Werte 1 bis 6 annimmt,

sowie deren physiologisch unbedenklichen Salze.

4) Verfahren zur Herstellung von Verbindungen der allgemeinen Formel (I)

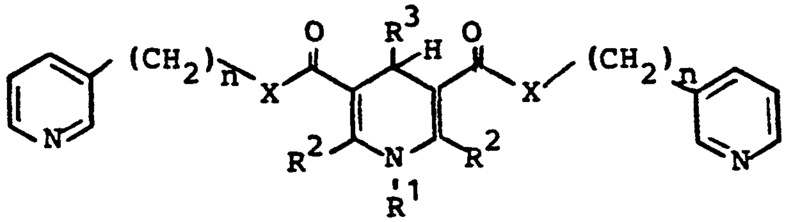

(I)

in der

$R^1$ Wasserstoff oder Alkyl (geradkettig oder verzweigt), gegebenenfalls substituiert durch Halogen, Nitro, Cyan, Alkoxy, Carboxy, Alkoxycarbonyl oder Carboxamid,

$R^2$ Wasserstoff oder Alkyl (geradkettig oder verzweigt),

$R^3$ Wasserstoff oder Alkyl (geradkettig oder verzweigt), Carboxy, Alkoxycarbonyl oder Aryl, gegebenenfalls substituiert durch Hydroxyalkyl, Carboxy, Sulfoxy, Acyloxyalkyl, Benzoyloxyalkyl,

X Sauerstoff, Schwefel oder NH bedeutet und

n die Werte 1 bis 6 annimmt,

sowie deren physiologisch unbedenklichen Salze,

dadurch gekennzeichnet, daß man Ketoverbindungen der allgemeinen Formel (II)

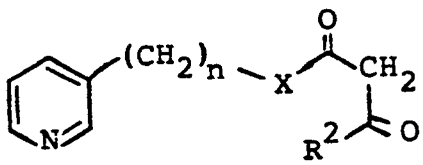

(II)

in welche

$R^2$, X und n die oben angegebene Bedeutung haben,

mit Aldehyden der allgemeinen Formel (III)

$$R^3-\underset{\underset{O}{\|}}{C}-H \qquad (III)$$

in welcher

$R^3$ die oben angegebene Bedeutung hat

und Aminen der allgemeinen Formel (IV)

$$R^1 - NH_2 \qquad (IV)$$

in welcher

0173204

$R^1$ die oben angegebene Bedeutung hat,

in Gegenwart eines inerten organischen Lösungsmittels umsetzt und gegebenenfalls in ihre physiologisch unbedenklichen Salze überführt.

5) Verfahren gemäß Anspruch 4, dadurch gekennzeichnet, daß man die Reaktion bei Temperaturen von 10°C bis 200°C durchführt.

6) Verfahren zur Herstellung der Verbindungen der allgemeinen Formel (I)

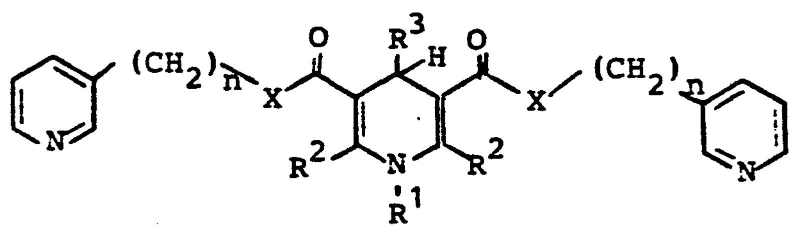 (I)

in welcher

$R^1$ Wasserstoff oder Alkyl (geradkettig oder verzweigt), gegebenenfalls substituiert durch Halogen, Nitro, Cyan, Alkoxy, Carboxy, Alkoxycarbonyl oder Carboxamid,

$R^2$ Wasserstoff oder Alkyl (geradkettig oder verzweigt),

$R^3$ einen durch die Gruppe $-\underset{\overset{\|}{O}}{C}-\underset{H}{N}-R^4$ substituierten Arylrest darstellt, wobei

$R^4$ für Alkyl (geradkettig oder verzweigt), Aryl, Heteroaryl, Aralkyl, Heteroalkyl oder die Gruppen 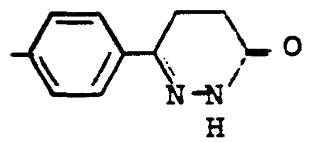 ,

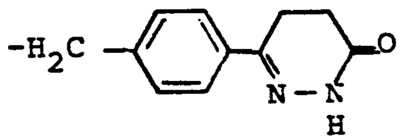

steht,

X Sauerstoff, Schwefel oder NH bedeutet und

n die Werte 1 bis 6 annimmt,

sowie deren physiologisch unbedenklichen Salze,

dadurch gekennzeichnet, daß man Verbindungen der allgemeinen Formel (I) in welcher

$R^1$, $R^2$, X und n die oben angegebene Bedeutung haben,

$R^3$ jedoch für 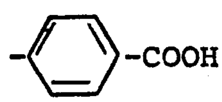 steht,

zunächst in aktivierte Carbonsäurederivate der allgemeinen Formel (V)

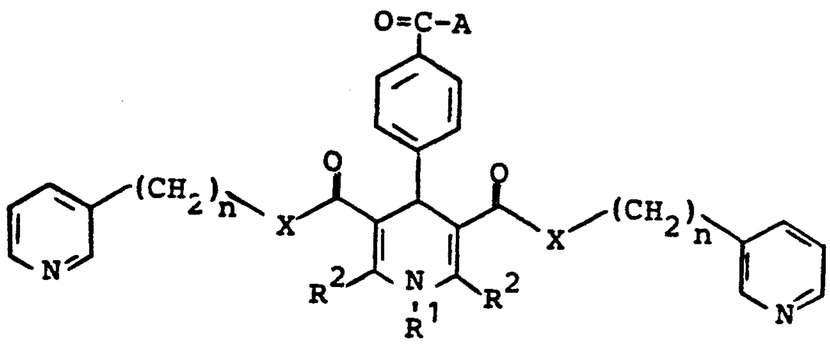 (V)

in welcher

$R_1$, $R_2$, X und n die oben angegebene Bedeutung haben und

A für einen elektronenziehenden Rest, wie er in der Peptidchemie zur Aktivierung von Carbonsäuren üblich ist, steht,

überführt und diese anschließend mit Aminen der allgemeinen Formel (VI)

$$H_2N\text{-}R^4 \qquad \text{(VI)}$$

in welcher

$R^4$ die oben angegebene Bedeutung hat,

in Gegenwart eines inerten organischen Lösungsmittels umsetzt und gegebenenfalls in ihre physiologisch unbedenklichen Salze überführt.

7) Verfahren gemäß Anspruch 6, dadurch gekennzeichnet, daß man die Reaktion bei Temperaturen von -70°C bis +60°C durchführt.

8) Arzneimittel enthaltend mindestens eine Verbindung der allgemeinen Formel (I) gemäß Anspruch 1.

9) Verwendung von Verbindungen der allgemeinen Formel (I) gemäß Anspruch 1 bei der Bekämpfung von thromboembolischen und ischämischen Erkrankungen.

10) Verfahren zur Herstellung von Arzneimitteln gemäß Anspruch 8, dadurch gekennzeichnet, daß man Verbindungen der allgemeinen Formel I gemäß Anspruch 1 gegebenenfalls unter Verwendung von üblichen Hilfs- und Trägerstoffen in eine geeignete Applikationsform überführt.